Europäisches Patentamt

European Patent Office

Office européen des brevets

(11) Publication number: **0 266 909**
**A1**

## (12) EUROPEAN PATENT APPLICATION

(21) Application number: 87308852.0

(22) Date of filing: 06.10.87

(51) Int. Cl.⁴: **B01J 13/02 , A61K 9/50**

(30) Priority: 06.10.86 US 915613
23.09.87 US 100850

(43) Date of publication of application:
**11.05.88 Bulletin 88/19**

(84) Designated Contracting States:
**CH DE ES FR GB IT LI NL SE**

(71) Applicant: **Midwest Research Institute**
**425 Volker Boulevard**
**Kansas City Missouri 64110(US)**

(72) Inventor: **Kuhn, Gustav O.**
**6900 W. 201st Terrace**
**Bucyrus Kansas(US)**
Inventor: **Engel, James F.**
**124 W. 78th Terrace**
**Kansas City Missouri(US)**

(74) Representative: **Lawrence, Peter Robin**
**Broughton et al**
**GILL JENNINGS & EVERY 53-64 Chancery**
**Lane**
**London WC2A 1HN(GB)**

(54) Method of micro-encapsulating chemicals.

(57) A process to produce microcapsules that meet the stringent requirements for toxicological studies involves the formation of an emulsion of the test chemical with a solution of a matrix material and spraying the emulsion through a nozzle (30) to form discrete masses of the emulsion. The masses travel through relatively dry, cool air (24 and 26) to partially solidify the matrix to yield individual microcapsules containing micelles (45) of the encapsulated substance distributed throughout the matrix. The microcapsules are received on a supporting surface (34) formed by a conveyor (32) covered with a layer of hydrophobic starch from a supply (40) and maintained separated from each other until the solidification of the matrix material has proceeded to a stage where the microcapsules do not have a tendency to stick together. The microcapsules are then received in a collector (42), separated from the starch and packaged.

Fig. 1.

## METHOD OF MICROENCAPSULATING CHEMICALS

This invention relates to a process for the microencapsulation of chemical substances. The invention has particular application for microencapsulation of test chemicals for toxicological studies.

Toxicology is the science of determining whether a test chemical has a deleterious physiological effect on living organisms. Toxicology studies involve exposing laboratory animals to test chemicals under carefully controlled conditions to determine if a test chemical is physiologically active to the animal, and if so, by what route of administration and at what dose level over what period of time. A number of different routes of administration of test chemicals are available to toxicologists and these include incorporation of the test chemical into the diet, drinking water, or breathing air of the animal or by direct application via injection, skin painting, and the like. The generally preferred route is via the test animal diet and it is this application which is a particular application of products of this invention.

Test chemicals may include any of a wide variety of chemical substances. Test chemicals may range from low moleular weight, volatile hydrocarbons, halocarbons, aldehydes, alcohols, ketones, amines, acids and the like to more complex materials such as antibiotics, hormones, vitamins, pesticides, herbicides, and similar materials. Generally, any material desired in microcapsule form would be a candidate for microencapsulation pursuant to the teachings of this invention. For toxicology testing, chemicals which may not be easily or advantageously admixed directly with the test animal diet are particular candidates for microencapsulation pursuant to this process.

Microencapsulation is a process used to envelop a small amount of a substance (the core) within a protective coating (the shell) which serves as a barrier to control or prevent loss of the substance through leakage, evaporation or reaction of the chemical with other materials to which it might be exposed. Microcapsules may consist of only one core within the shell or may contain a number of discrete or substantially discrete subunits or micelles of core chemical dispersed within a matrix of shell material. This latter form of microencapsulation is the subject of this invention.

Microencapsulation has been used for many years to provide, for example, small units of materials such as flavors, fragrances, or inks, and for controlled-release pharmaceutical dosage forms, and for timed-release pesticides, fertilizers, and insect repellants in the field of agriculture, and other products.

Microencapsulation has heretofore been tried as a means of preparing test chemicals for administration to laboratory animals for toxicology studies. Potentially physio logically active substances prepared in this manner can be handled much more safely than in other, more conventional forms. However, toxicologists are concerned that additional substances other than the test chemical and diet nutrients not be introduced to the animals so that the results of the study can be properly interpreted. A drawback has been encountered with the use of heretofore available processes for microencapsulation. The microcapsules produced by prior processes tend to contain undesirable residues of catalysts, hardening agents, emulsifiers, solvents, unreacted monomers or the like which are likely to obfuscate the test results. Alternatively or additionally, some existing encapsulation processes produce microcapsules with shells or matrices which are not easily digested by the test animals, leading to questionable bioavailability of the test chemical. In addition, the matrix material must not promote constipation, diarrhea or other long-term digestive disorders in the test animals. Many matrix materials used in conventional microencapsulation processes would produce such side effects if administered to the test animal over an extended period of time as is required for toxicology studies.

Another disadvantage of conventional microencapsulation processes is the inability to achieve the desired quantities of the core material in the microcapsule, especially when volatile or reactive chemicals are utilized as the core material. Spray driers are generally utilized in these processes to cure or dry the matrix material and the elevated temperatures in these driers makes them particularly unsuitable for microencapsulating volatile or reactive chemicals.

Accordingly, it is a primary object of this invention to provide a method whereby substances, and particularly physiologically active, volatile or chemical reactive substances, may be encapsulated to provide microcapsules capable of meeting the substantial constraints established for toxicology studies, but suitable also for other purposes.

In such a method, in accordance with the invention, an emulsion is formed with micelles of the core material dispersed throughout a liquid matrix material which, when intended for use in toxicology studies, needs to be capable of being readily digested in the stomach of a human or a test animal; the emulsion is then pressurized and discharged through an orifice to generate a spray of discrete, relatively small masses of the emulsion which are moved through an unsupported path of travel while simultaneously being exposed to a conditioning medium to at least partially solidify the matrix material of the masses and are then collected whereby the masses comprise microcapsules with quantities of the core material dispersed as micelles throughout the matrix material the microcapsules comprising approximately 30 to 70% core material by weight.

Such a method can provide a process wherein the microcapsules have shells or matrices providing substantially nonpermeable barriers to prevent leakage or evaporation of volatile chemicals and further to protect the test chemical in the core from chemical reaction with atmospheric chemicals or other substances to which it might be exposed. Of course, the process of microencapsulation must not alter the chemistry of the test chemical.

The method may involve a process wherein the shell or matrix materials are of non-toxic food grade materials that are capable of rapid dissolution in the test animal after ingestion and which may be fed to the test animals over an extended period of time without producing undesirable side effects. In such a microencapsulation process, the sizes of the microcapsules may be appropriate to permit homogeneous mixtures with the feed for the test animals and to prevent the animals from differentiating between the microcapsules and the feed.

In the process the resulting microcapsules may be sufficiently durable to resist the abrasion which inevitably occurs during the process of mixing the microcapsules with the feed for the test animals. The percentage of the test chemical contained in the core of the microcapsule may be relatively high to permit, for example, substantial doses of the encapsulated test chemical to be administered to the test animals without the necessity of administering such amounts of the shell or matrix material that could result in significant changes in nutrient contents of the diets of the test animals or undesirable side effects.

The invention will be further explained or will become apparent from the following description and explanation. In the drawing:

Figure 1 is a schematic illustration of equipment which may be used in carrying out the process of this invention;

Fig. 2 is a fragmentary, vertical cross-sectional view through a nozzle used in making microcapsules pursuant to this invention;

Fig. 3 is an enlarged plan view of the mixing element of the nozzle of Fig. 2 and taken along line 3-3 of Fig. 2; and

Fig. 4 is a fragmentary, greatly enlarged cross-sectional view through a typical microcapsule produced by the method of this invention.

The microencapsulation process of this invention is carried out by forming an emulsion of the test chemical with a solution of the shell or matrix material. An emulsion consists of a discontinuous phase, in this case the test chemical, which is dispersed throughout a continuous phase, in this case a liquid form or a solution of the shell or matrix material. While the sizes of units of the discontinuous phase may vary, it would normally be desired to be about 0.1 to about 50 microns and preferably from 0.1 to 10 microns.

The discontinuous phase comprises the material to be encapsulated. It may be a liquid form or could be a solution or a dispersion, for example, of micronized solids in a liquid. The discontinuous phase may also be a solid in particulate form having sizes as necessary for appropriate dispersal in the matrix material.

The continuous phase of the emulsion is, of course, the material which provides the matrix to encapsulate the discontinuous phase. A variety of materials can be used as the matrix material, it being understood that the particular matrix material chosen to carry out the microencapsulation process must be compatible for the purpose with the particular core material involved. The objective is to produce microcapsules of particular sizes containing desired quantities of the core material in substantially unmodified form as an end product. Accordingly, the matrix material chosen would ordinarily be one which does not chemically react with the core material and one in which the core material would be formulated to suppress the solubility of the core material in the matrix material. Otherwise, the core material would have a tendency to permeate the matrix or shell and dissipate.

It is also necessary to choose a matrix material which is readily digestable in the stomach of the test animal and which may constitute up to 5% of the test animal diet for the lifetime of the animal without promoting side effects such as constipation, diarrhea and other digestive disorders.

Suitable matrix materials for microencapsulation pursuant to this process often are proteins such as gelatin, albumin, zein, and the like. Depending, of course, on the nature of the core material to be encapsulated, other matrix or shell materials such as modified or unmodified food grade starches may be utilized. Because of the particular constraints imposed by toxicology studies, materials such as high melting fats, waxes, vegetable gums, and substituted cellulose derivatives are generally unacceptable as matrix materials. The matrix material may require additives to decrease the permeability of the core material or to increase the plasticity of the matrix or to achieve other desired properties. Additives which have been found to be useful with certain formulations include sugars, starches, dextrins, polyols, mineral salts, amino acids and/or their salts and the like.

The matrix material must be in a liquid state to form the emulsion with the core material. Frequently, solutions containing the materials mentioned above are utilized, with water being the most common solvent especially for toxicology studies, but the use of other solvents would be obvious to those skilled in the art.

A high yield of encapsulated material is desired, that is, a high percentage of the discontinuous phase of the emulsion should be incorporated into usable product microcapsules. This is especially true for test chemicals for toxicology testing. The typical test chemical is often expensive and frequently available in only limited supply. Further, since the test chemical is suspected of being physiologically active, it must be contained and not released to the environment. Thus, yields of acceptable microcapsules from the process must be as high as practicable. In addition, since the only useful product for such studies is that which falls within the acceptable sizes specified in accordance with the intended use, the process should be capable of producing a very high percentage of microcapsules within the desired size limits. While the range of sizes selected may vary, for toxicology testing it would normally be about a 100 to 400 micron range.

Additionally, as heretofore pointed out, a microcapsule shell or matrix should provide low permeability for the core or test chemical. Tentative criteria established as acceptable for microcapsules for toxicology studies specify that for volatile chemicals the microcapsules retain 99 plus percentage of the chemical load after one month of storage under optimum conditions (generally in closed containers at room temperature). The permeation of low volatility chemicals through the shell or matrix of microcapsules containing such chemicals is evaluated by washing the microcapsules with a solvent in which the core material is soluble, but which does not dissolve the shell or matrix material. Satisfactory microcapsules will typically indicate less than one percent of chemical load removable by solvent washing.

From the foregoing, it will be understood that the choice of materials for producing microcapsules pursuant to this process involves formulation in accordance with the requirements specified for the end product and the chemical properties of the core chemical. The selection of appropriate formulations of materials to produce microcapsules of particular substances by following the process of this invention is, of course, within the ability of those skilled in the art.

Once a suitable matrix material has been chosen to be used with the particular core material to be encapsulated, an emulsion is formed of the core material within the matrix material liquid. Emulsification may be carried out by any suitable means, for example, mechanical stirring with an appropriate stirrer blade at an appropriate speed to produce shear and cavitation forces sufficient to disperse the core material within the matrix liquid. The extent of emulsification will, of course, depend upon the nature of the particular materials involved, the desired size range for the dispersed phase, and the percentage loading of core material desired within the microcapsules. While the percentage loading may vary, for toxicology studies it normally would be desired to have loadings as high as practicable, with realistic loadings for this purpose being from within a low of 30 percent to a high of 70 percent range.

Once the emulsion has been formed, it is pressurized and discharged through a spray nozzle to generate discrete masses or droplets. Since it is desirable to avoid mixing air with the emulsion to avoid foaming and/or oxidation of the core material during pumping, the emulsion is pumped by a mechanical spraying device. A high speed vibratory atomization device of this type which is capable of producing pressures in the order of about 50 psi to 100 psi (345 to 690 KN/M²) has been found effective for this purpose. The emulsion is discharged through an orifice at this pressure to produce the spray pattern of individual masses or droplets. An orifice of 0.4 millimeter diameter has been found effective to produce microcapsules having sizes of about 75 microns. An orifice of 0.8 millimeter diameter has produced microcapsules of up to 500 microns. The particular size orifice to be used to form the spray droplets in accordance with this process depends on the sizes desired for the resulting microcapsules.

The production from the spray nozzle of droplets of the sizes desired not only depends on orifice size, but may also depend on pressure and viscosity of the discharged material. These factors can be varied as required for the materials involved as will be readily apparent to those skilled in the art.

In order to maintain the integrity of the discrete masses of sprayed material, the spray is preferably directed generally horizontally into air which has been conditioned to provide for a certain amount of drying and partial solidification of the masses before they gravitate to a supporting surface. The temperature and humidity of the air should be carefully controlled to provide optimum curing conditions for the microcapsules during this phase of the process.

It is desired to minimize volatilization of the core material while maximizing evaporation of water from the aqueous phase of the emulsion. Alternatively, the air should be cold enough to facilitate congealing of a shell or matrix material such as gelatin. Empirical tests have found that air having a temperature of from about 18°C to about 28°C and having a relative humidity of from about 15 percent to about 45 percent is suitable for these purposes. The particles emanating from the spray orifice preferably travel through this conditioned air medium about 6 , feet (1800 mm) horizontally and about 6 feet down to a catching or collecting surface. This amount of unsupported travel through the conditioned air medium has been found to allow the microcapsules to at least partially solidify before coming to rest.

The individual particles of emulsion which leave the spray orifice in droplet form inherently assume spherical or near spherical shapes during the unsupported travel through the air. This, of course, is a phenomenon resulting from the action of the surface tension forces on the droplet during its unsupported travel. Solidification of the matrix. material around the core material during unsupported travel through the air produces individual microcapsules.

In order to prevent the partially cured microcapsules from sticking together at this stage of production, they are allowed to fall on a layer of hydrophobic starch. The starch serves to keep the discrete masses or microcapsules separated until the curing or congealing of the shell material has proceeded to a point where the capsules no longer exhibit a tendency to stick to one another. This may involve an interval of from about 1 to 2 minutes duration for most materials used in this process.

A starch layer about two millimeters thick is considered adequate for receiving microcapsules ranging in size from about 100 to 400 microns and sprayed for gravitation through a conditioned air medium as set forth above. Sufficient starch must be provided to maintain the individual integrity of the particles until they have hardened sufficiently. On the other hand, excessive starch beyond that needed for this purpose complicates the step of separating the starch from microcapsules to attain the desired end product.

Once the microcapsules have solidified sufficiently (i.e. usually about one to two minutes after coming to rest in the starch) they are separated from the starch. The presently preferred method of separation is by the use of mechanical sifting, but other acceptable means could also be used. The separated microcapsules are then typically conditioned in ambient air for a further period of from several hours to as long as several days before they are considered ready for packaging as may be desired.

Using the described process in a series of trial runs, 100 gram to 150 gram batches of microcapsules were made from trichloroethylene and an aqueous solution of gelatinsorbitol (70:30, 25% solids). The mixtures (55:45, w/w) were emulsified in a variety of ways to afford homogeneous emulsions with micelle sizes ranging, step wise, from 0.1 to 10 microns in a series of batches. The core material loadings ranged from 35% to 45% in the final microcapsules. The temperature and relative humidity of the conditioning air were approximately 25°C and 20%, respectively, during all runs. The yields of microcapsules of acceptable sizes (from 100 to 450 microns) exceeded 90% in all runs.

In another series of trial runs, 100 gram to 300 gram batches of microcapsules were made from 2-ethylhexanol and an aqueous solution of Capsul® starch, 40% solids. The mixtures (50:50, w/w) were emulsified to afford homogeneous emulsions with micelle sizes ranging from approximately 1 micron to 15 microns, step wise, in a series of batches. The core material loadings ranged from 35% to 55% in the batches. The temperature and relative humidity of the conditioning air were approximately 25°C and 15%, respectively, during all runs. The yields of microcapsules of acceptable sizes (from 100 to 450 microns) exceeded 90% in all runs.

The applicability of the described process to the microencapsulation of low boiling, water insoluble organic liquids has been demonstrated with numerous chemicals. Representative of this class of compounds, a series of 400 g batches of microcapsules was prepared using trans-1,2-dichloroethylene (B.P. 47°C) and an aqueous solution of a modified food grade starch and sucrose (80:20 w/w, 40% solids). The mixtures, ranging from 60:40 w/w to 48:52 w/w, were emulsified with a standard Ross high shear emulsifier at temperatures ranging from 14 to 22°C. Emulsions containing micelles ranging from approximately 0.5 to 2 microns were sprayed into conditioned air at 26°C and 39% relative humidity. The final dried microcapsules contained from 51.5 to 39.7% chemical by weight. After 28 days exposure in open dishes at ambient temperature and humidity conditions, the samples retained 97 to 98% of their zero time chemical loads.

Chemicals which are readily oxidizable in air are among the most difficult to microencapsulate successfully by pre-existing technology. By using the present process, chemicals of this type have been successfully encapsulated for use in toxicology studies. As an example, an approximately 1-kg batch of microcapsules was prepared using trans-cinnamaldehyde and an aqueous 40% w/w solids solution of modified food grade starch and sucrose. (Cinnamaldehyde is readily oxidized to trans-cinnamic acid in air.). The mixture of chemical and shell solution (55:45 w/w) was emulsified at 42°C under an argon atmosphere to obtain micelles ranging from 0.5 to 2 microns. The emulsion was then sprayed into conditioned air at 27°C and 37% relative humidity. The final dried microcapsules contained approximately 44% chemical load, and chemical analysis indicated only 0.5% increase in cinnamic acid content over that which was present in the starting chemical. Other readily oxidizable chemicals such as citral and refined fish oils have similarly been encapsulated by the process.

An additional example of the versatility of the described process is the microencapsulation of a series of volatile, low molecular weight silicone oils. Approximately 600 g batches of microcapsules were made using Dow Corning silicone oils Nos. 200, 244, and 245, and an aqueous solution of gelatin, sucrose, and high-dextrin corn syrup (80:15:5 w/w, 16% solids). The mixtures (approximately 55:45% w/w) were emulsified at 45°C and sprayed into conditioned air at 26°C and 40% relative humidity. The final dried microcapsules contained approximately 35% chemical by weight and exhibited 99% retention of chemical load after 28 days exposure to ambient temperature and humidity conditions in open dishes.

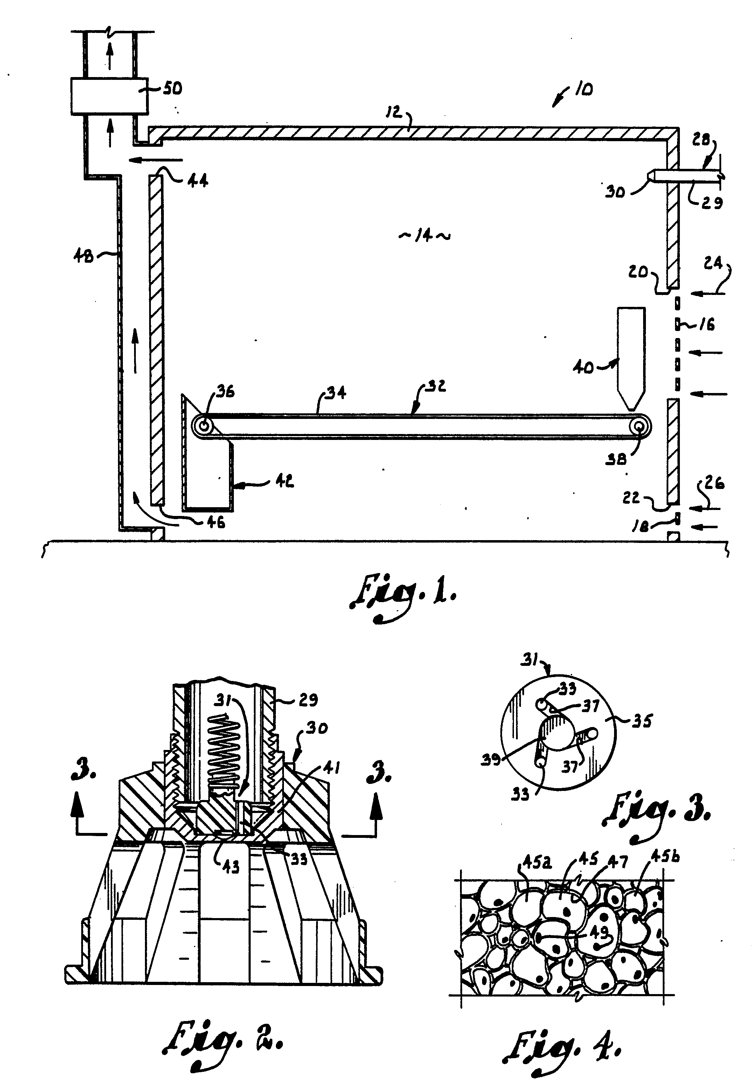

Apparatus for carrying out the process of this invention in a continuous manner is illustrated - schematically in Figure 1 of the drawing. The apparatus is broadly designated by the reference numeral 10 and includes a substantially closed housing 12 defining a chamber 14 to protect the environment against the inadvertent escape of test chemical substances and the like, and to provide a controlled environment for the production of microcapsules. Grills 16 and 18 across inlet openings 20 and 22 respectively in one end of housing 12 permit the flow of separate streams 24 and 26 of air into chamber 14, either or both of such streams being susceptible for preconditioning as to temperature and humidity or both by suitable apparatus (not shown) prior to introduction into the chamber.

A sprayer 28 projects through the wall of housing 12 as shown and terminates in a nozzle 30 positioned generally near the top and at one end of chamber 14 in position to produce a spray pattern generally horizontally across the chamber. An endless belt type conveyor 32 extends horizontally in chamber 14 beneath nozzle 30 in disposition to receive the particles emanating from nozzle 30 as the latter gravitate toward the bottom of the chamber. Conveyor 32 comprises an endless belt 34 which is preferably of fiberglass construction hav-

ing a Teflon® coating on the outer surface. Belt 34 is trained over spaced apart rollers 36 and 38, one or both of which is powered by a motor (not shown) to drive the belt in a continuous manner with the upper surface of the belt proceeding from the right to the left as viewed in Fig. 1.

Apparatus broadly designated by the numeral 40 is provided for continuously applying a uniform layer of hydrophobic starch to the upper surface of the belt and across the width of the latter at the upstream or right end of the belt as viewed in Fig. 1. A·collector 42 is provided at the downstream or discharge end of the belt to receive the starch and microcapsules produced by this process. Collector 42 might advantageously include apparatus (not shown) such as sifters or the like for continuously separating the microcapsules from the starch material and for conveying the microcapsules and the starch separately to their respective desired destinations.

Outlet openings 44 and 46 in the end of housing 12 opposite that containing inlet openings 20 and 22 communicate with a vertically extending duct 48. Air egressing from chamber 14 through openings 44 and 46 is conducted by duct 48 through a filter 50 before discharging to the atmosphere. Filter 50 may be of any suitable type capable of removing materials from the air stream which may have become entrained during the microencapsulation process and which would be dangerous or objectionable for discharging into the environment.

The emulsion is discharged through nozzle 30 at a pressure which may be on the order of about 50 to 100 psi (345 to 690 KN/M²) for some materials. Pressures in this range have been found satisfactory for producing discrete masses of the emulsion and to project them along a trajectory involving a path of travel extending about six feet (1800 mm) in the horizontal direction and about six feet downwardly to conveyor 32.

The air entering through grill 16 is preconditioned to the desired temperature and relative humidity heretofore described. If desired or required, the air entering through grill 18 can also be preconditioned. In any event, the discrete masses or particles in the spray pattern should be exposed to air conditioning to a sufficient degree to effect a drying or partial solidification of the particles as they gravitate toward the conveyor.

A conveyor 32 having a length of approximately 20 feet (6100 mm) and a width of approximately 4 feet (1200 mm) has been found acceptable for the purpose of carrying out the microencapsulation process of this invention. The speed of travel of the conveyor is about 12 feet (3600 mm) per minute, thereby providing about two minutes total travel time for the microcapsules received on the belt before they are deposited into collector 42. This time allows for further curing or solidification of the separate microcapsules while they are maintained in a separated condition by the starch on the belt.

Studies involving electron photomicrographs of microcapsules produced by the method of this invention reveal that the microcapsules typically contain a very high number of very small micelles. Most micelles are of less than two microns in size and exist as readily identifiable, spheroid masses separated from adjacent micelles by extremely thin membranes or micelle walls comprised of matrix material.

While the respective individual micelles are substantially discrete, in the sense that the major chamber of each micelle appears to be separate and distinct from the major chambers of the other micelles, the studies reveal that many of the micelles are actually in communication with one or more adjacent micelles by means of microscopic openings through the micelle walls.

It is believed that the composition of the microcapsules where they are comprised of a vast number (up to about 100,000,000) of very small (most $\leq$ 1 micron) micelles accounts for the very high loading which applicants have been able to achieve. It is evident that the nature of the micelle structure for the microcapsules results from the degree of emulsification of the materials. Some of this emulsification occurs during the stirring step but it is evident that further emulsification occurs during the spraying of the materials through the nozzle. The emulsification which occurs at the nozzle is thought to be important, and perhaps highly significant to the satisfactory production of microcapsules with loadings of core material within the specified range. It is believed that the added dispersal of the dispersed phase, which occurs as the emulsion is forced through the nozzle immediately prior to discharge through the controlled atmosphere, results in the production of microcapsules containing such a great number of extremely small micelles.

Figures 2 and 3 of the drawing illustrates a nozzle which has been found acceptable for producing the required shear in the emulsion pumped through the nozzle at the pressures mentioned so that the micelle sizes are about two microns and less. The nozzle 30 includes a mixing element 31 having a plurality of longitudinally extending conduits 33 for conducting the emulsion from pipe 29 to the face 35 of the element. Each conduit 33 has a groove 37 in the face 35 extending from its respective conduit to a circular, central cavity 39 in the element face. The grooves 37 are positioned to feed generally tangentially into cavity 39.

Element 31 fits against the inner surface of a disc section of a nozzle nose cap 41 as shown in Fig. 2. Material pumped through element 31 and into the chamber defined by cavity 39 and the nose cap is discharged through an orifice 43 aligned with the cavity. It is believed that the mechanical action given the emulsion when forced through a nozzle of this general type contributes to the production of an emulsion wherein the micelles of dispersed material are extremely small and uniformly dispersed throughout the matrix material.

Figure 4 of the drawing is an illustration of a representative cross-section through a microcapsule produced by the method of this invention. Figure 4 is taken from a photomicrograph at 7500X magnification. The substantially discrete micelles 45 are separated from adjacent micelles by micelle walls 47 comprising extremely thin membranes of matrix material. Some micelles such as 45a and 45b are completely isolated by the micelle walls from other micelles. On the other hand, many of the micelles are in communication with one or more adjacent micelles through openings 49 through the micelle walls.

The process described herein for the production of microcapsules containing test chemicals as may be utilized in toxicology studies, has proven extremely effective. Attempts to use more conventional systems of microencapsulation such as coacervation have not met with success, particularly in the field of microencapsulation for toxicology studies because of the contamination of the core chemical by residues from the encapsulation process. Heretofore available spray drying techniques have been tried with equally unsuccessful results because of the inability to incorporate sufficient amounts of the core material, especially volatile materials, in the capsules by the use of such techniques. Microcapsules utilized in the flavor industry, for example, typically contain no more than a maximum of 20 percent of core material. The minimum amount of core material contained in a microcapsule acceptable for many toxicology studies is on the order of approximately 40 percent. Utilizing the process of this invention, applicants have been able to obtain the encapsulation of approximately 60 percent of the core material with certain emulsion formulations.

While the process of this invention has been described particularly with respect to the production of microcapsules of materials for toxicological studies, the process may also be used for other advantageous purposes. For instance, the process may be utilized with suitable core materials to produce microcapsules which may be used as feed additives for animals or food additives for human consumption. The scope of this disclosure and the appended claims should be considered to include the encapsulation of chemicals generally, wherein test chemicals for toxicological studies is but one very important example.

## Claims

1. A process for producing microcapsules comprising a core material contained within a protective matrix, in which process an emulsion is formed with micelles (45) of the core material dispersed throughout a liquid matrix, the emulsion is pressurized and discharged through an orifice (43) to generate a spray of discrete, relatively small masses of the emulsion which are moved through an unsupported path of travel while simulta-neously being exposed to a conditioning medium (24,26) to at least partially solidify the matrix material of the masses and are then collected, whereby the masses conprise microcapsules with quantities of the core material dispersed as micelles (45) throughout the matrix material, the microcapsules comprising approximately 30 to 70% core material by weight..

2. A process as set forth in claim 1, wherein the emulsion is formed by mechanically stirring a mixture comprised of the core material and the matrix material with sufficient agitation to effect shearing and cavitation.

3. A process as set forth in claim 1 or claim 2, wherein the micelles (45) of core material in the emulsion have sizes of less than about 50 microns.

4. A process as set forth in claim 3, wherein the sizes of micelles (45) or core material in the emulsion fall within a range of 0.1 micron to 10 microns.

5. A process as set forth in claim 3, wherein the size of the micelles (45) of core material in the emulsion fall within a range of 1 micron to 50 microns.

6. A process as set forth in any one of the preceding claims, in which the sizes of the micelles are reduced by passing the pressurized emulsion throughout an atomizing nozzle (30) prior to discharging the emulsion through the orifice (43).

7. A process as set forth in claim 6, wherein substantially all of the micelles (45) are no larger than about 2 microns.

8. A process as set forth in any one of the preceding claims wherein the liquid matrix is capable of being readily digested in the stomach of a human or a test animal.

9. A process as set forth in any one of the preceding claims, wherein the liquid matrix material is a protein solution.

10. A process as set forth in any one of claims 1 to 8, wherein the liquid matrix material is an aqueous solution of starch.

11. A process as set forth in any one of the preceding claims, wherein the core material comprises a low boiling, water insoluble organic liquid.

12. A process as set forth in any one of claims 1 to 10 wherein the core material comprises a volatile, low molecular weight silicone oil.

13. A process as set forth in any one of claims 1 to 10, wherein the core material comprises a dispersion of micronized solids in a liquid.

14. A process as set forth in any one of claims 1 to 10, wherein the core material comprises solids in particulate form.

15. A process as set forth in any one of the preceding claims, wherein the sizes of the masses generated by forcing the emulsion under pressure through a spray nozzle (30) are within the range of 100 microns to 400 microns in diameter.

16. A process as set forth in any one of the preceding claims, wherein the conditioning medium (24,26) is air.

17. A process as set forth in claim 26, wherein the air (24,26) is conditioned to a temperature within a range of 18°C to 28°C.

18. A process as set forth in claim 16 or claim 17, wherein the air is conditioned to a relative humidity within a range of 15% to 45%.

19. A process as set forth in any one of the preceding claims, wherein the masses are received on a supporting surface (34) following the step of moving the masses through the conditioning medium (24,26) and wherein the masses, at least partially solidified, are maintained with each mass physically separated from the other masses for a predetermined interval of time to permit further solidification of the matrix material while supported by the surface (34).

20. A process as set forth in claim 19, wherein the predetermined time interval is from about one to about two minutes.

21. A process as set forth in claim 19 or claim 20, wherein the exposure of the masses to the conditioning medium (24,26) is maintained while the masses are supported on the surface (34) during the predetermined time interval.

22. A process as set forth in any one of claims 19 to 21, wherein the supporting surface (34) is provided with a layer of hydrophobic starch prior to receiving the masses, whereby the starch aids in maintaining the masses in the physically separated condition during solidification of the matrix material.

23. A process as set forth in claim 22, wherein the layer of starch on the surface (34) is about 2 mm thick.

0 266 909

Fig. 1.

Fig. 2.

Fig. 3.

Fig. 4.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 070 719 (UNILEVER) <br> * Page 6, lines 9-36; page 7, lines 1-28; page 3, lines 33-35 * <br> --- | 1,2,10, 11,19, 20,22 | B 01 J 13/02 <br> A 61 K 9/50 |
| X | FR-A-1 287 716 (PHILIPS' GLOEILAMPENFABRIEKEN) <br> * Page 3, left-hand column, paragraph 3, right-hand column, paragraph 3; page 4, left-hand column, paragraph 3 * <br> --- | 1,2,8,9 ,15,19 | |
| X | US-A-4 286 020 (C.M. HIMEL et al.) <br> * Column 2, lines 6-50; column 3, lines 58-68; column 8, lines 24-41 * <br> --- | 1,3,4,5 ,7,14, 15,16 | |
| X | FR-A-1 259 081 (HOFFMANN-LA ROCHE) <br> * Page 2, left-hand column, paragraph 3; page 2, right-hand column, paragraphs 1,2 * <br> --- . | 1,2,8,9 ,14,16 | |
| A | EP-A-0 152 285 (SOUTHWEST RESEARCH INSTITUTE) <br> * Page 5, lines 20-37; page 6, lines 1-19; page 2, lines 21-30 * <br> --- | 1,11,16 ,19,21, 22 | TECHNICAL FIELDS SEARCHED (Int. Cl.4) <br><br> B 01 J <br> B 41 M <br> A 61 K |
| A | FR-A-1 281 523 (VITAMINS LTD) <br> * Page 2, left-hand column, paragraph 3; page 6, left-hand column, paragraph 2, right-hand column, paragraph 4 * <br> ----- | 1,6 | |

The present search report has been drawn up for all claims

| Place of search | Date of completion of the search | Examiner |
|---|---|---|
| THE HAGUE | 15-01-1988 | KERRES P.M.G. |